(19) Europäisches Patentamt
European Patent Office
Office européen des brevets

(11) Veröffentlichungsnummer: **0 163 672 B1**

(12) **EUROPÄISCHE PATENTSCHRIFT veröffentlicht nach Art. 158 Abs. 3 EPÜ**

(45) Veröffentlichungstag der Patentschrift: **15.05.91**

(51) Int. Cl.$^5$: **C07D 307/935**, A61K 31/557, //C07D309/12,C07C405/00

(21) Anmeldenummer: **84904100.9**

(22) Anmeldetag: **08.11.84**

(86) Internationale Anmeldenummer: **PCT/DE84/00238**

(87) Internationale Veröffentlichungsnummer: **WO 85/02187 (23.05.85 85/12)**

Die Akte enthält technische Angaben, die nach dem Eingang der Anmeldung eingereicht wurden und die nicht in dieser Patentschrift enthalten sind.

# (54) 20-ALKYL-7-OXOPROSTACYCLINDERIVATE UND VERFAHREN ZU IHRER HERSTELLUNG.

(30) Priorität: **10.11.83 DE 3340988**

(43) Veröffentlichungstag der Anmeldung: **11.12.85 Patentblatt 85/50**

(45) Bekanntmachung des Hinweises auf die Patenterteilung: **15.05.91 Patentblatt 91/20**

(84) Benannte Vertragsstaaten: **AT BE CH DE FR GB LI LU NL SE**

(56) Entgegenhaltungen:
**WO-A-82/01002**

**Prostaglandins, Band 16, Nr. 6, Dez 1978, Los Altos, Calif.(US) D.A.van DORP et al. : "20-Methyl-prostacyclin a powerful 'unnatural' platelet aggregation inhibitor", Seiten 953-955**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **NICKOLSON, Robert**
**Fuchsienweg 12 c**
**W-1000 Berlin 65(DE)**
Erfinder: **VORBRÜGGEN, Helmut**
**Wilkestr. 7**
**W-1000 Berlin 27(DE)**
Erfinder: **STÜRZEBECHER, Claus - Steffen**
**Brigittenstrasse 6a**
**W-1000 Berlin 46(DE)**
Erfinder: **HABEREY, Martin**
**Neckarsulmer Str. 15**
**W-1000 Berlin 46(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 163 672 B1

## Beschreibung

Die Erfindung betrifft neue Prostacyclin-Derivate, Verfahren zu ihrer Herstellung sowie ihre Verwendung als Arzneimittel.

Prostacyclin ($PGI_2$), einer der Hauptfaktoren bei der Blutplättchenaggregation, wirkt dilatierend auf verschiedene Blutgefäße (Science 196, 1072) und könnte daher als Mittel zur Blutdrucksenkung in Betracht komme . $PGI_2$ besitzt jedoch nicht die für ein Arzneimittel notwendige Stabilität. So beträgt seine Halbwertszeit bei physiologischen pH-Werten und bei Raumtemperatur nur wenige Minuten.

In DE-A 30 35 454 werden chemisch stabile und biologisch wirksame 7-Oxoprostacycline beschrieben. Aus WO 82/01002 sind 7-Oxoprostacycline mit ungesättigten Alkylresten in 18- und 19-Stellung bekannt. Unter den namentlich gennanten Beispielen findet sich jedoch kein Beispiel mit einer 20-Alkyl-Substitution. Durch die Publikation von D.A. von Dorp et al. in Prostaglandins 16, 953 (1978) ist außerdem bekannt, daß 20-Methylprostacyclin 2-3 mal stärker Thrombozytenaggregations-hemmend wirkt als Prostacyclin selbst.

Es wurde gefunden, daß die Einführung einer Alkylgruppe in 20-Stellung des Prostacyclins zu einer weiteren Wirkungssteigerung der 7-Oxoprostacycline führt, wobei das pharmakologisch Wirkungsspektrum erhalten bleibt und die Wirkungsdauer der neuen Prostacycline deutlich verlängert wird.

Die erfindungsgemäßen Verbindungen inhibieren die Thrombozytenaggregation und wirken blutdrucksenkend und bronchodilatatorisch. Sie wirken außerdem cytoprotektiv an Herz, Leber, Magen und Niere und hemmen die Magensäuresekretion.

Die Erfindung betrifft 7-Oxoprostacycline der allgemeinen Formel I

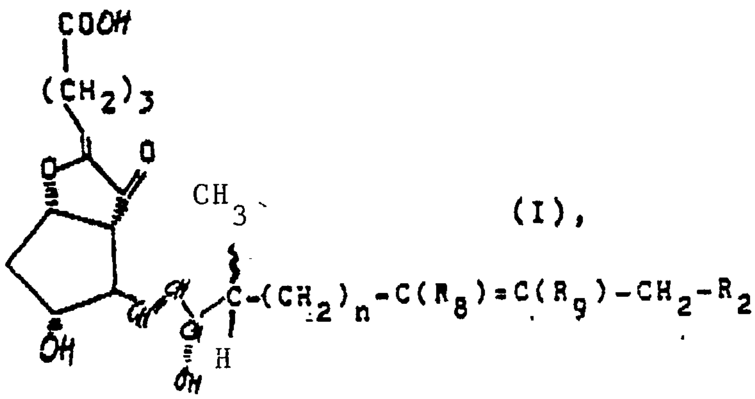

(I),

worin

| | |
|---|---|
| n | 1 oder 2 |
| $R^2$ | Wasserstoff, wenn n = 2 bedeutet, oder eine Methylgruppe, wenn n = 1 bedeutet, |
| $R^8$ und $R^9$ | gemeinsam eine Bindung oder Wasserstoff bedeuten und deren Salze mit physiologisch verträglichen Basen. |

Zur Salzbildung mit den freien Säuren sind anorganische und organische Basen geeignet, wie sie dem Fachmann zur Bildung physiologisch verträglicher Salze bekannt sind.

Beispielsweise seien genannt: Alkalihydroxide, wie Natrium- und Kaliumhydroxid, Erdalkalihydroxide, wie Calciumhydroxid, Ammoniak, Amine, wie Äthanolamin, Diäthanolamin, Triäthanolamin, N-Methylglucamin, Morpholin, Tris(hydroxymethyl)-methylamin usw..

Die Erfindung betrifft ferner ein Verfahren zur Herstellung der erfindungsgemäßen 7-Oxoprostacycline der allgemeinen Formel I dadurch gekennzeichnet, daß man in an sich bekannter Weise eine Verbindung der allgemeinen Formel II

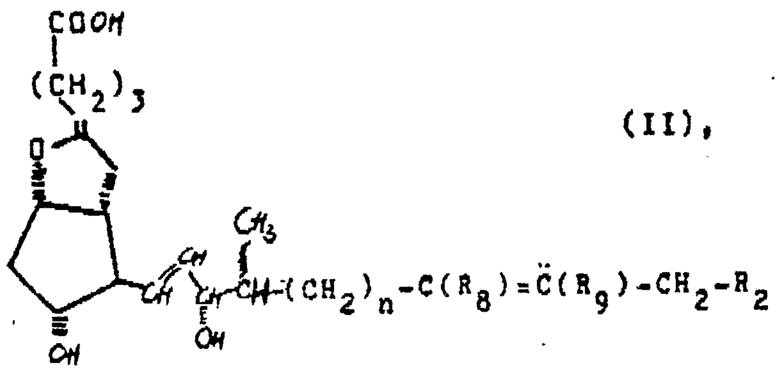

(II),

worin n, $R^2$ $R^8$ und $R^9$ die oben angegebenen Bedeutungen aufweisen, mit Selendioxid oxydiert.

Die Umsetzung der Verbindung der allgemeinen Formel II mit Selendioxid wird bei Temperaturen von 20-140° C, vorzugsweise bei 50-120° C, in einem organischen Lösungsmittel, vorzugsweise Dioxan oder tert.-Butanol in 0,5-10 Stunden unter Inertgas (wie z.B. $N_2$ oder Ar) und unter Rühren gegebenenfalls unter Zusatz einer Amin-Base, wie Pyridin oder Hexamethyldisilazan vorgenommen.

Die 7-Oxoprostacyclin-Derivate der allgemeinen Formel I können mit geeigneten Mengen der entsprechenden anorganischen Basen unter Neutralisierung in Salze überführt werden. Beispielsweise erhält man beim Lösen der entsprechenden PG-Säuren in Wasser, welches die stöchiometrische Menge der Base enthält, nach Abdampfen des Wassers oder nach Zugabe eines mit Wasser mischbaren Lösungsmittels, zum Beispiel Alkohol oder Aceton, das feste anorganische Salz.

Zur Herstellung eines Aminsalzes, die in üblicher Weise erfolgt; wird die Prostacyclin-Säure zum Beispiel in einem geeigneten Lösungsmittel, beispielsweise Äthanol, Aceton, Acetonitril, Diäthyläther oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die funktionelle Abwandlung der freien OH-Gruppen erfolgt nach den dem Fachmann bekannten Methoden. Zur Einführung der Ätherschutzgruppen wird beispielsweise mit Dihydropyran in Methylenchlorid, Benzol oder Chloroform unter Verwendung eines sauren Katalysators, wie zum Beispiel $POCl_3$, p-Toluolsulfonsäure oder wasserfreier Mineralsäuren umgesetzt. Das Dihydropyran wird im Überschuß angewandt, vorzugsweise in der 2- bis 10-fachen Menge des theoretischen Bedarfs. Die Umsetzung ist normalerweise bei 0° C bis 30° C nach 15-30 Minuten beendet.

Die Einführung der Acylschutzgruppen erfolgt, indem man eine Verbindung der allgemeinen Formel I in an sich bekannter Weise mit einem Carbonsäurederivat, wie zum Beispiel Säurechlorid,Säureanhydrid u.a., in Gegenwart einer tertiären Aminbase, wie z.B. Pyridin, Dimethylaminopyridin etc. umsetzt.

Die Freisetzung einer funktionell abgewandelten OH-Gruppe zu den Verbindungen der allgemeinen Formel I erfolgt nach bekannten Methoden. Beispielsweise wird die Abspaltung der Ätherschutzgruppen in einer wässrigen Lösung einer organischen Säure, wie zum Beispiel Essigsäure, Propionsäure u.a. oder in einer wässrigen Lösung einer anorganischen Säure, wie zum Beispiel Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind zum Beispiel Alkohole, wie Methanol und Äthanol und Äther, wie Dimethoxyäthan, Dioxan und Tetrahydrofuran. Tetrahydrofuran wird bevorzugt angewendet. Die Abspaltung wird vorzugsweise bei Temperaturen zwischen 20° C und 80° C durchgeführt.

Die Abspaltung der Silylätherschutzgruppen erfolgt beispielsweise mit Tetrabutylammoniumfluorid oder mit KF in Gegenwart eines Kronenäthers. Als Lösungsmittel sind beispielsweise geeignet Tetrahydrofuran, Diäthyläther, Dioxan, Methylenchlorid usw. Die Abspaltung wird vorzugsweise bei Temperaturen zwischen 0° C und 80° C durchgeführt.

Die Verseifung der Acylgruppen erfolgt beispielsweise mit Alkali- oder Erdalkali-carbonaten oder -hydroxyden in einem Alkohol oder der wässrigen Lösung eines Alkohols. Als Alkohole kommen aliphatische Alkohole in Betracht, wie zum Beispiel Methanol, Äthanol, Butanol usw., vorzugsweise Methanol. Als Alkalicarbonate und -hydroxyde seien Kalium- und Natriumsalze genannt, bevorzugt sind jedoch die Kaliumsalze. Als Erdalkalicarbonate und -hydroxyde sind beispielsweise geeignete Calciumcarbonat, Calciumhydroxyd und Bariumcarbonat.Die Umsetzung erfolgt bei -10° C bis 70° C, vorzugsweise bei 25° C.

Die als Ausgangsmaterial dienenden Verbindungen der allgemeinen Formel II können beispielsweise hergestellt werden, indem man in an sich bekannter Weise ein bekanntes Prostaglandin F-Derivat der

allgemeinen Formel III

(III),

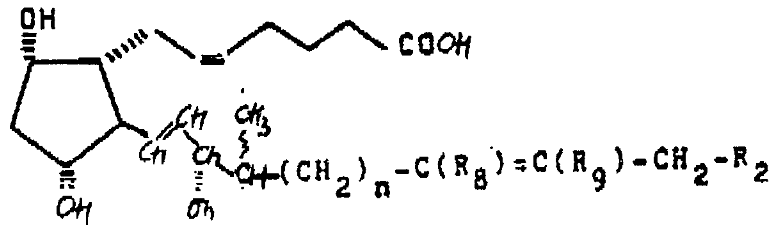

mit Jod in Gegenwart eines Alkalihydrogencarbonats oder Alkalicarbonats zu den Verbindungen der allgmeinen Formel IV

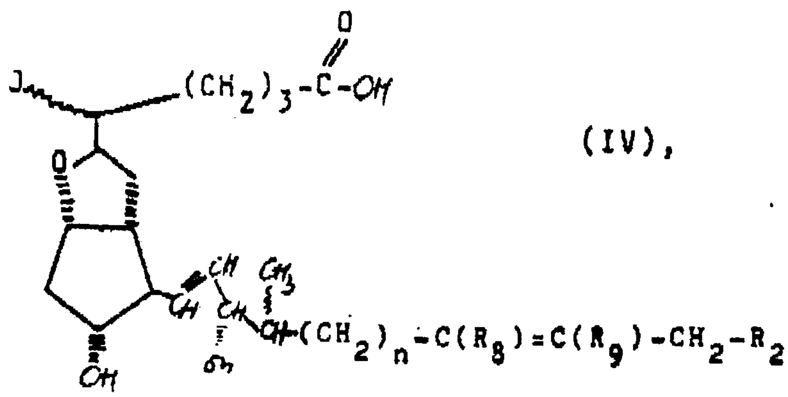

(IV),

umsetzt [J. Tomosközi et al., Tetrahedron Letters, 2627 (1977)].

Anschließend kann man gegebenenfalls freie Hydroxygruppen durch Veresterung, Verätherung oder Silylierung schützen.

Die Umsetzung der Verbindungen der allgemeinen Formel IV zu den Verbindungen der allgemeinen Formel II kann beispielsweise mit 1,5-Diazabicyclo[3,4,0]nonen-5(DBN) oder 1,5-Diazabycyclo[5,4,0)-undecen-5(DBU) in einem inerten Lösungsmittel wie Benzol, Toluol, Tetrahydrofuran usw. oder mit Natriummethylat in Methanol erfolgen. Die Halogenwasserstoffabspaltung wird bei Temperaturen zwischen 0° C und 120° C, vorzugsweise bei 20-60° C durchgeführt.

Weil die Endprodukte freie Hydroxylgruppen im Prostanrest enthalten, geht man zweckmäßigerweisevon Ausgangsprodukten aus, in denen diese durch vorzugsweise leicht abspaltbare Äther- oder Acylreste intermediär geschützt sind.

Die Verbindungen dieser Erfindung wirken blutdrucksenkend und bronchodilatorisch. Sie sind weiterhin geeignet zur Hemmung der Thrombozyten-Aggregation. Folglich stellen die neuen Prostacyclin-Derivate der Formel I wertvolle pharmazeutische Wirkstoffe dar. Darüber hinaus weisen sie bei ähnlichem Wirkungsspektrum, verglichen mit entsprechenden Prostaglandinen aus EP 59756, eine höhere Spezifität und vor allem eine wesentlich längere Wirksamkeit auf. Im Vergleich zu $PGI_2$ zeichnen sie sich durch größere Stabilität aus. Die hohe Gewebsspezifität der neuen Prostaglandine zeigt sich bei der Untersuchung an glattmuskulären Organen, wie z.B. am Meerschweinchenileum oder an der isolierten Kaninchentrachea, wo eine wesentlich geringere Stimulation zu beobachten ist als bei der Applikation natürlicher Prostaglandine vom E., A- oder F-Typ.

Die neuen Prostaglandin-Analoga besitzen die für Prostacycline typischen Eigenschaften, wie z.B. Senkung des peripheren arteriellen und koronaren vaskulären Widerstandes, Inhibierung der Thrombozytenaggregation und Auflösung von Plättchenthromben, myocardiale Zytoprotektion und damit Senkung des systemischen Blutdruckes ohne zugleich Schlagvolumen und koronare Durchblutung zu senken; Behandlung von Schlaganfall, Prophylaxe und Therapie koronarer Herzerkrankungen, koronarer Thrombose, des Herinfarkts, peripherer Arterienerkrankungen, Arteriosklerose und Thrombose, Therapie des Schocks, Inhibierung der Bronchokonstriktion, Inhibierung der Magensäuresekretion und Zytoprotektion der Magen- und Darmschleimhaut; antiallergische Eigenschaften, Senkung des pulmonaren vaskulären Widerstandes und des pulmonaren Blutdruckes, Förderung der Nierendurchblutung, Anwendung an Stelle von Heparin oder

als Adjuvans bei der Dialyse der Hämofiltration, Konservierung von Blutplasmakonserven, besonders von Blutplättchenkonserven, Inhibierung von Geburtswehen, Behandlung von Schwangerschaftstoxikose, Erhöhung der zerebralen Durchblutung etc.. Außerdem besitzen die neuen Prostaglandinanaloga antiproliferative Eigenschaften.

Die Dosis der Verbindungen ist 1-1 500 µg/kg/Tag, wenn sie am menschlichen Patienten verabreicht werden. Die Einheitsdosis für den pharmazeutisch akzeptablen Träger beträgt 0,01-100 mg.

Bei intravenöser Injektion an wachen, hypertonen Ratten in Dosen von 5, 20 und 100 µg/kg Körpergewicht zeigen die erfindungsgemäßen Verbindungen eine stärkere blutdrucksenkende und länger anhaltende Wirkung als $PGE_2$ und $PGA_2$, ohne wie $PGE_2$ Durchfälle oder $PGA_2$ kardiale Arrhythmien auszulösen.

Bei intravenöser Injektion an narkotisierten Kaninchen zeigen die erfindungsgemäßen Verbindungen in Vergleich zu $PGE_2$ und $PGA_2$ eine stärkere und erheblich länger anhaltende Blutdrucksenkung, ohne daß andere glattmuskuläre Organe oder Organfunktionen beeinflußt werden.

Für die parenterale Verabreichung werden sterile, injizierbare, wässrige oder ölige Lösungen benutzt. Für die orale Applikation sind beispielsweise Tabletten, Dragees oder Kapseln geeignet. Für die topische Anwendung sind Salben, Cremes, Gele und Lösungen geeignet.

Die Erfindung betrifft damit auch Arzneimittel auf Basis der Verbindungen der allgemeinen Formel I und üblicher Hilfs- und Trägerstoffe.

Die erfindungsgemäßen Wirkstoffe sollen in Verbindung mit den in der Galenik bekannten und üblichen Hilfsstoffen, z.B. zur Herstellung von Blutdrucksenkern dienen.

Beispiel 1

16,20-Dimethyl-7-oxo-18,18,19,19-tetradehydro-$PGI_2$

1a) (1S,5R,6R,7R)-6-[(E)-(4RS)-3-Oxo-4-methyl-non-1-en-6-inyl]-7-benzoyloxy-2-oxabicyclo[3.3.Q]octan-3-on

Eine Lösung von 6,84 g Dimethyl-(2-oxo-3-methyl-oct-5-inyl)phosphonat in 50ml wasserfreiem Dimethoxyäthan tropft man zu einer Suspension von 1,21g Natriumhydrid/Ölsuspension (55%ig) in 60ml Dimethoxyäthan bei Raumtemperatur. Danach wird 30 Minuten bei Raumtemperatur gerührt und dann auf -30° abgekühlt. Man tropft nun eine Lösung von 6,93 g Corey-Lacton-Aldehyd in 75 ml Dimethoxyäthan zu, rührt 1 Stunde bei -30° und 1 1/2 Std. bei -15°. Durch Zugabe von 11 ml Eisessig wird die Reaktion abgebrochen. Man verdünnt mit Diäthyläther und neutralisiert durch Schütteln mit gesättigter Natriumhydrogencarbonatlösung. Die organische Phase wird mit gesättigter Natriumchloridlösung gewaschen, mit Magnesiumsulfat getrocknet und nach Filtrieren im Vakuum zur Trockne eingeengt. Man erhält 11,86 g der Titelverbindung. Die Ausbeute nach Reinigung beträgt 98,6% der Theorie.

IR: 1760 $cm^{-1}$ (3-on), 1720 $cm^{-1}$ (7-benzoat), 1700 $cm^{-1}$ (3-oxo)

1b) (1S,5R,6R,7R)-6-[(E)-(3RS,4RS)-3-Hydroxy-4-methyl-non-1-en-6-inyl]-7-benzoyloxy-2-oxabicyclo[3.3.0]-octan-3-on

11,8 g Rohprodukt der nach 1a) hergestellten Verbindung löst man in 400 ml wasserfreiem Methylalkohol, kühlt auf -40° ab und fügt 6,59g Natriumborhydrid zu. Nach 20 Minuten werden vorsichtig 11,6 ml Eisessig zugetropft. Man läßt auf Raumtemperatur erwärmen und dampft dann das Lösungsmittel im Vakuum bei maximal 30° ab. Der Rückstand wird mit Methylenchlorid versetzt. Die organische Phase wird je einmal mit Wasser, 3%iger Natriumhydrogencarbonatlösung und halbgesättigter Natriumchloridlösung gewaschen. Man trocknet mit Magnesiumsulfat, filtriert und engt das Filtrat im Vakuum zur Trockne ein. Die so erhaltenen 11,65 g Rohprodukt werden zur Trennung der 3R- und 3S-Isomeren an Kieselgel mit einem Pentan-Diäthyläther-Gradientensystem chromatographiert. Man erhält neben 250 mg Ausgangsverbindung 5,04 g des S-Isomeren, entspr. 51% der Theorie und 4,44 g des R-Isomeren = 45% der Theorie. Durch Rückoxydation des R-Isomeren (Jones-Reagenz in Aceton) und Wiederholung der Reaktion kann die Ausbeute am S-Isomeren auf über 75% gesteigert werden.

IR: 3460 $cm^{-1}$ (3-Hydroxy), 1760 $cm^{-1}$ (3-on), 1720 $cm^{-1}$ (7-benzoat)

1c) (1S,5R,6R,7R)-6[(E)-(3S,4RS)-3-Hydroxy-4-methyl-non-1-en-6-inyl]-7-hydroxy-2-oxabicyclo[3.3.0]octan-3-on

7,03g der Verbindung gemäß Beispiel 1b) (S-Isomeres) löst man in 65ml wasserfreiem Methylalkohol, fügt 1,05g wasserfreies Kaliumcarbonat zu und rührt unter Argon und Feuchtigkeitsausschluß 3 Stunden bei Raumtemperatur. Dann wird durch Zugabe halbkonzentrierter Salzsäure neutralisiert und der Methylalkohol im Vakuum bei einer Badtemperatur von max. 30° abdestilliert. Der Rückstand wird in Dichlormethan gelöst und mit Magnesiumsulfat getrocknet. Der nach Abfiltrieren des Trockenmittels und Entfernen des Lösungsmittels verbleibende Rückstand wird durch Chromatographier an Kieselgel mit einem Dichlormethan/Aceton-System gereinigt. Man erhält 4,23 g entspr. 81,6 % der Theorie.

IR: 3350 $cm^{-1}$ (Dihydroxy), 1760 $cm^{-1}$ (3-on)

1d) (1S,5R,6R,7R)-6-[(E)-(3S,4RS)-4-Methyl-3-(tetrahydropyran-2-yloxy)-non-1-en-6-inyl]-7-(tetrahydropyran-2-yloxy)-2-oxabicyclo[3.3.0]octan-3-on

2,10g der nach Beispiel 1c hergestellten Verbindung löst man in 65ml getrocknetem Dichlormethan, gibt 1,54 ml Dihydropyran und 10 mg p-Toluolsulfonsäure-monohydrat zu und rührt 2 Stunden bei Raumtemperatur. Die Reaktionslösung wird je einmal mit eiskalter verdünnter Natriumhydrogencarbonatlösung und eiskalter Natriumchloridlösung gewaschen, mit Magnesiumsulfat getrocknet und im Vakuum eingeengt. Der Rückstand wird durch Chromatographie an Kieselgel mit einem Hexan/Äthylacetat-System gereinigt. Man erhält 2,96 g der Titelverbindung entsprechend 89,5% der Theorie.

IR: 1760 $cm^{-1}$ (3-on), 1295, 870 und 815 $cm^{-1}$ (Tetrahydropyranyläther)

1e) (1S,3RS,5R,6R,7R)-6-[(E)-(3S,4RS)-4-Methyl-3-(tetrahydropyran-2-yloxy)-non-1-en-6-inyl]-7-(tetrahydro pyran-2-yloxy)-2-oxabicyclo[3.3.0]octan-3-ol

2,96 g der nach Beispiel 1d hergestellten Verbindung löst man in 75 ml absolutem Toluol. Unter Argonüberleitung kühlt man auf -70° C ab und tropft 11,2 ml einer 20%igen Lösung von Diisobutylaluminiumhydrid in Toluol zu. Nach 30 Minuten zerstört man das überschüssige Hydrid durch Zutropfen von 0,72ml Isopropylalkohol. Nun wird das Kühlbad entfernt, der Ansatz mit 5,56 ml Wasser versetzt und solange gerührt, bis die Aluminiumverbindungen absaugbar sind. Es wird mit Dichlormethan nachgewaschen und das Filtrat im Vakuum zur Trockne eingeengt. Die Titelverbindung fällt in quantitativer Ausbeute an.

IR: 1295 $cm^{-1}$, 870 $cm^{-1}$ und 815 $cm^{-1}$ (Tetrahydropyranyläther) 3400 $cm^{-1}$ (3-ol)

1f) 11,15-Bis-(tetrahydropyran-2-yloxy)-16,20-dimethyl-18,18,19,19-tetradehydro-$PGF_{2\alpha}$

Bei 0° tropft man zu einer Mischung von 24,2 ml Hexamethyldisilazan und 70 ml frisch von Lithiumaluminiumhydrid destilliertem Tetrahydrofuran 92ml einer 1,25 molaren Lösung von n-Butyllithium in Hexan. Man rührt noch 15 Minuten und tropft dann diese Lithiumsilazidlösung zu einer Suspension von 25,6 g Carboxybutyltriphenylphosphoniumbromid in 295ml absolutem Tetrahydrofuran. Wenn die Umsetzung zum Ylid beendet ist, wird eine Lösung von 3,15 g der nach Beispiel le hergestellten Verbindung in 200 ml absolutem Tetrahydrofuran zugegeben und der Ansatz 2 Stunden auf 40-45° erwärmt. Dann fällt man in eiskalter 10%iger Natriumchloridlösung, säuert durch Zugabe von 10%iger Zitronensäurelösung auf PH 4 an und extrahiert fünfmal mit Diäthyläther. Die vereinigten Extrakte werden nun viermal mit eiskalter 2,5%iger Natronlauge extrahiert. Der alkalische Extrakt wird mit 10%iger Zitronensäurelösung angesäuert und erneut fünfmal mit Diäthyläther extrahiert. Der Extrakt wird mit Magnesiumsulfat getrocknet, filtriert und im Vakuum eingeengt. Der Rückstand wird roh weiterverarbeitet.

1g) 11,15-Bis-(tetrahydropyran-2-yloxl)-16,20-dimethyl)-18,18,19,19-tetradehydro-$PGF_{2\alpha}$-methylester

5,22 g des nach Beispiel 1f erhaltenen Rohprodukts werden in 125 ml Dichlormethan gelöst und auf 0° abgekühlt. Nun gibt man ätherische Diazomethanlösung zu, bis ein geringer Überschuß vorhanden ist, läßt 15 Minuten nachreagieren und zerstört dann den Überschuß durch einige Tropfen Essigsäure. Der nach

Abdestillieren des Lösungsmittels verbleibende Rückstand wird durch Chromatographie an Kieselgel mit einem Hexan/Aceton-System gereinigt. Man erhält 2,72 g des Methylesters, das sind 71,2% der Theorie, bezogen auf das Lactol gemäß Beispiel 1e.

IR: 3450 $cm^{-1}$ (9-OH), 1730 $cm^{-1}$ (Methylester)

1h) 11,15-Bis-(tetrahydropyran-2-yloxy)-16,20-dimethyl)-5-jod-18,18,19,19-tetradehydro-$PGI_1$-methylester

Zu einer Lösung von 2,68 g des nach Beispiel 1g erhaltenen Methylesters in 60ml Diäthyläther gibt man eine Lösung von 5,89 g Natriumhydrogencarbonat in 85 ml Wasser. Unter kräftigem Rühren wird auf 0° abgekühlt und eine Lösung von 2,53 g Jod in 35 ml Diäthyläther tropfenweise zugefügt. Es wird 2 Stunden bei 0° nachgerührt. Man gibt die Reaktionslösung in einen Scheidetrichter, trennt die Phasen und wäscht die ätherische Phase einmal mit 5%iger Natriumthiosulfatlösung und zweimal mit Wasser. Die vereinigten Wasserphasen werden mit Diäthyläther nachextrahiert. Die organische Phase wird mit Magnesiumsulfat getrocknet. Der nach Abfiltrieren und Einengen erhaltene Rückstand wird durch Chromatographier an Kieselgel mit einem Hexan/Aceton-System gereinigt. Ausbeute 3,13 g, entsprechend 95% der Theorie.

IR: 1730 $cm^{-1}$ (Methylester)

1i) 11,15-Bis-(tetrahydropyran-2-yloxy)-16,20-dimethyl-18, 18,19,19-tetradehydro-$PGI_2$-methylester

483 mg des nach 1h erhaltenen Methylesters werden in 7 ml wasserfreiem Benzol gelöst, mit 1,54 ml Diazabicycloundecen versetzt und unter Argon 2,5 Stunden bei 55-60° gerührt. Nach dem Abkühlen auf Raumtemperatur wird mit Äthylacetat verdünnt und dreimal mit Wasser gewaschen. Der wäßrige Extrakt wird einmal mit Äthylacetat reextrahiert und die organische Phase mit Magnesiumsulfat getrocknet. Der nach Filtrieren und Abdampfen der Lösungsmittel erhaltene Rückstand wird als Rohprodukt weiterverarbeitet.

1j) 7-Oxo-11,15-bis-(tetrahydropyran-2-yloxy)-16,20-dimethyl-18,18,19.19-tetradehydro-$PGI_2$-methylester

145 mg des nach 1i) erhaltenen Rohprodukts löst man in 5 ml Dioxan. Nach Zugabe von o,o5 ml Hexamethyldisilazan und 41 mg frisch sublimiertem Selendioxid rührt man unter Argon eine Stunde bei 100°. Man läßt auf Raumtemperatur abkühlen und gießt die Reaktionsmischung in basisches Eiswasser. Dann extrahiert man alternierend je zweimal mit Diäthyläther und Äthylacetat, wäscht die vereinigten Extrakte einmal mit Wasser und trocknet die organische Phase mit Magnesiumsulfat. Darauf wird filtriert und im Vakuum zur Trockne eingeengt. Der Rückstand wird durch präparatuve Dünnschichtchromatographie an Kieselgelplatten im System Hexan/Äthylacetat/Triäthylamin (7/3/0,5) getrennt. Man erhält 48 mg an roher Titelverbindung, aus welchen nach nochmaliger Reinigung im System Dichlormethan/Diäthyläther (85/15) 36 mg Reinprodukt erhalten werden Neben der Titelverbindung erhält man noch 45 mg rohes 5-Oxo-11,15-bis-(tetrahydropyran-2-yloxy)-16,20-dimethyl-6,7-dehydro-18,18,19,19-tetradehydro-$PGI_1$-methylester, Ausbeute an Titelverbindung bezogen auf den Methylester nach Beispiel 1h beträgt 29,8% der Theorie.

IR: 1730$cm^{-1}$ (7-Oxo- und Methylester), 1650 $cm^{-1}$ (5-en)

1k) 7-Oxo-16,20-dimethyl-18,18,19,19-tetradehydro-$PGI_2$-methylester

415 mg der nach Beispiel 1j hergestellten 7-Oxa-Verbindung versetzt man mit 11ml einer Mischung aus 65 Teilen Eisessig, 35 Teilen Wasser und 10 Teilen Tetrahydrofuran und rührt unter Argon 24 Stunden bei Raumtemperatur. Die Hauptmenge der Essigsäure wird im Vakuum bei Raumtemperatur abdestilliert. Der Rest an Essigsäure wird durch 2maliges Abdestillieren mit Toluol entfernt. Der Rückstand wird durch Chromatographie an Kieselgel mit einem Hexan/Aceton-System gereinigt. Man erhält 246 mg der Titelverbindung, entsprechend 84% der Theorie.

IR: 346O $cm^{-1}$ (11,15-diol), 1730 $cm^{-1}$ (Methylester), 1700 $cm^{-1}$ (Schulter) (7-Oxo), 1650 $cm^{-1}$ (5-en)

1l) 7-Oxo-16,20-dimethyl-18,18,19,19-tetradehydro-$PGI_2$

221 mg der nach 1 k hergestellten Verbindung werden in 8ml Methylalkohol gelöst. Man gibt 1,55 ml einer Lösung von 200 mg Kaliumhydroxid in 2,5 ml Wasser und rührt unter Argon 20 Stunden bei Raumtemperatur. Der Methylalkohol wird im Vakuum bei Raumtemperatur abdestilliert und der Rückstand in Wasser aufgenommen. Man extrahiert 3mal mit Diäthyläther. Die wäßrige Phase wird durch Zugabe von 10%iger Zitronensäurelösung angesäuert und erneut 3mal mit Diäthyläther und einmal mit Äthylacetat extrahiert. Der Extrakt wird mit Magnesiumsulfat getrocknet, filtriert und im Vakuum von den Lösungsmitteln befreit. Das erhaltene Rohprodukt wird durch präparative Dünnschichtchromatographie im System Dichlormethan/ Methylalkohol (8/2) gereinigt. Man erhält 114 mg reines 16,20-Dimethyl-7-oxo-18,18,19,19-tetradehydro-$PGI_2$, das sind 53,4% der theoretischen Ausbeute.

IR: 3400 $cm^{-1}$ (breit)(11,15-Diol + Säure-OH), 1710 $cm^{-1}$ (breit) (Säure + 7-Oxo), 1650 $cm^{-1}$ (5-en)

## Beispiel 2

16,20-Dimethyl-7-oxo-19,19,20,20-tetradehydro-$PGI_2$

2a) (1S,5R,6R,7R)-6-[(E)-(4RS)-3-Oxo-4-methyl-1-nonen-7-inyl]-7-benzoyloxy-2-oxabicyclo[3.3.0]octan-3-on

Eine Lösung von 7,56 g Dimethyl-2-oxo-3-methyl-6-octinylphosphonat in 60 ml Dimethoxyethan wird zu einer Suspension vcn 1 ,33 g Natriumhydrid (55%ig in Öl) in 65 ml Dimethoxyethan bei Raumtemperatur. Danach wird die Lösung 30 Minuten bei Raumtemperatur gerührt und auf -30° C abgekühlt. Anschließend wird bei -20° C eine Lösung von 7,66 g (1S,5R,6R,7R)-6-Formyl-7-benzoyloxy-2-oxabicyclo [3.30] octan-3-on in 84 ml Dimethoxyethan zugegeben und das Gemisch wird weitere 2 Stunden bei Raumtemperatur gerührt. Das Rohprodukt wird ananlog Beispiel 1a) isoliert. Man erhält 12,95 g der Titelverbindung als öl.

IR: 1760 $cm^{-1}$, 1720 $cm^{-1}$, 1700 $cm^{-1}$, 1640 $cm^{-1}$, 1460 $cm^{-1}$.

2b) (1S,5R,6R,7R)-6-[(E)-(3S,4RS)-3-Hydroxy-4-methyl-1-nonen-7-inyl]-7-hydroxy-2-oxabicyclo[3.3.0]octan-3-on

Analog von Beispielen 1b)- 1c) erhält man 4,82 g der Titelverbindung ausgehend von 12,88 g des in Beispiel 2 a) hergestellten Ketons.

IR: 3600, 2965, 1770, 975 $cm^{-1}$.

2c) 16,20-Dimethyl-7-oxo-19,19,20,20-tetradehydro-$PGI_2$

Analog den Beispielen 1d) - 1l) werden 143 mg der Titelverbindung ausgehend von 4,70 g des in Beispiel 2b) hergestellten Diols erhalten.

IR: 3400 (breit), 1710, 1650, 1460, 1360 $cm^{-1}$.

## Beispiel 3

16,20-Dimethyl-7-oxo-18,19-didehydro-$PGI_2$

3a) (1S,5R,6R,7R)-6- [(1E,6Z)-(3S,4RS)-3-Hydroxy-4-methyl-1,6-nonadienyl]-7-hydroxy-2-oxabicyclo[3.3.0]-octan-3-on

Eine Lösung von 5,20 g der nach Beispiel 1c) hergestellten Acetylen-Verbindung in 170 ml Tetrahydrofuran wird mit 1,7 g Lindlar-Katalysator versetzt und 60 Minuten unter einer Wasserstoff-Atmosphäre (Normaldruck) gerührt. Nach dieser Stunde wqr die Wasserstoff-Aufnahme beendet und die Suspension

wurde vom Katalysator durch Filtrieren getrennt. Das Filtrat wurde im Vakuum zur Trockne eingeengt. Man erhält 5,22 g der Titelverbindung.

IR: 3350, 1760 $cm^{-1}$.

3b) 16,20-Dimethyl-7-oxo-18,19-didehydro-$PGI_2$

Analog den Beispielen 1d) - 1l) werden 171 mg der Titelverbindung ausgehend von 5,10 g des in Beispiel 3a) hergestellten Diols erhalten.

IR: 3400 (breit), 1710, 1650, 1460, 1360 $cm^{-1}$.

Beispiel 4

16,20-Dimethyl-7-oxo-19,20-didehydro-$PGI_2$

Analog den Beispielen 3a) - 3b) werden 160 mg der Titelverbindung ausgehend von 4,82 g des nach Beispiel 2b) hergestellten Diols erhalten.

IR: 3400 (breit), 1710, 1640, 1350 $cm^{-1}$.

## Ansprüche

**1.** 20-Alkyl-7-oxoprostacyclinderivate der allgemeinen Formel I

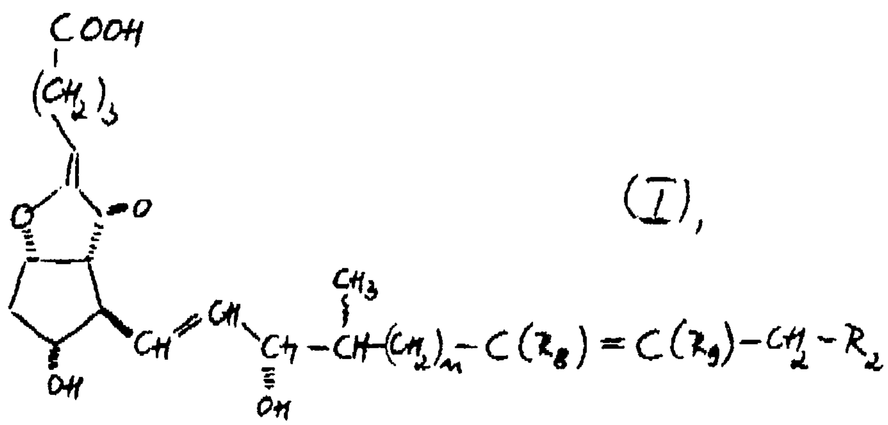

(I),

worin
n 1 oder 2,
$R^2$ Wasserstoff, wenn n = 2 bedeutet, oder eine Methylgruppe, wenn n = 1 bedeutet,
$R^8$ und $R^9$ gemeinsam eine Bindung oder Wasserstoff bedeuten
und deren Salze mit physiologisch verträglichen Basen.

**2.** Arzneimittel, bestehend aus einer oder mehreren Verbindungen gemäß Anspruch 1 und üblichen Hilfs- und Trägerstoffen.

**3.** Verfahren zur Herstellung von 7-Oxoprostacyclinen der allgemeinen Formel I, dadurch gekennzeichnet, daß man in an sich bekannter Weise eine Verbindung der allgemeinen Formel II,

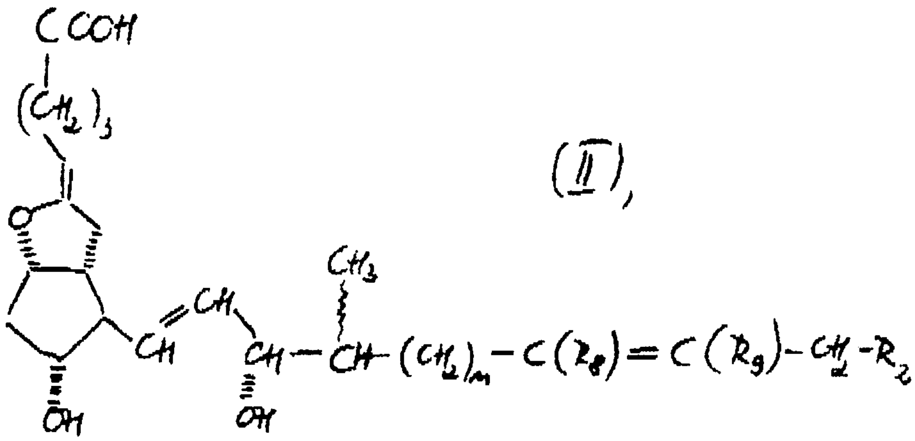

(II),

worin n, $R_2$, $R_8$ und $R_9$ die obenangegebene Bedeutung aufweisen, mit Selendioxid oxydiert.

## Claims

1. 20-alkyl-7-oxoprostacycline derivatives of the general formula I

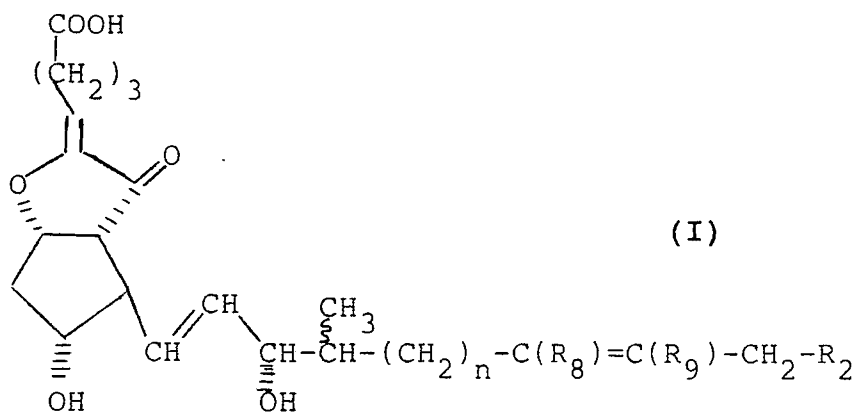

(I)

in which
n is 1 or 2,
$R^2$ is hydrogen when n = 2 or a methyl group when n = 1,
$R^8$ and $R^9$ together represent a bond or hydrogen,
and salts thereof with physiologically tolerable bases.

2. Medicament comprising one or more compounds according to claim 1 and customary adjuncts and carriers.

3. Process for the preparation of 7-oxoprostacyclines of the general formula I, characterised in that a compound of the general formula II

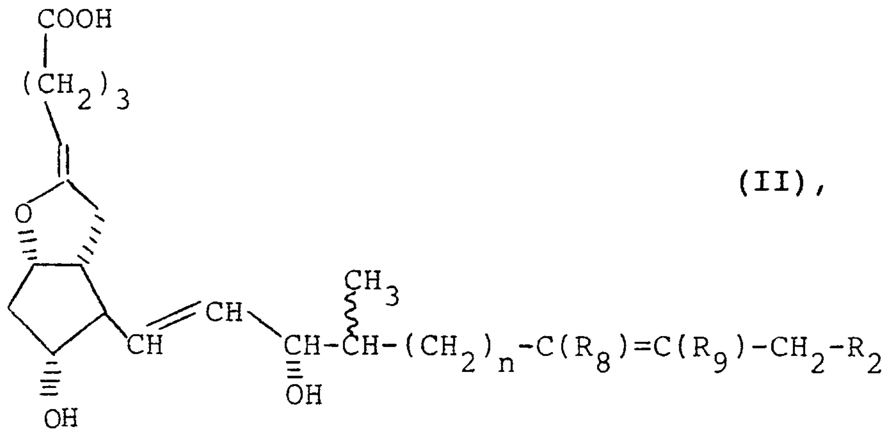

(II),

wherein n, $R_2$, $R_8$ and $R_9$ have the meanings given above, is oxidised in a manner known *per se* with selenium dioxide.

## Revendications

1. Alkyl-20 oxo-7 prostacyclines répondant à la formule générale I :

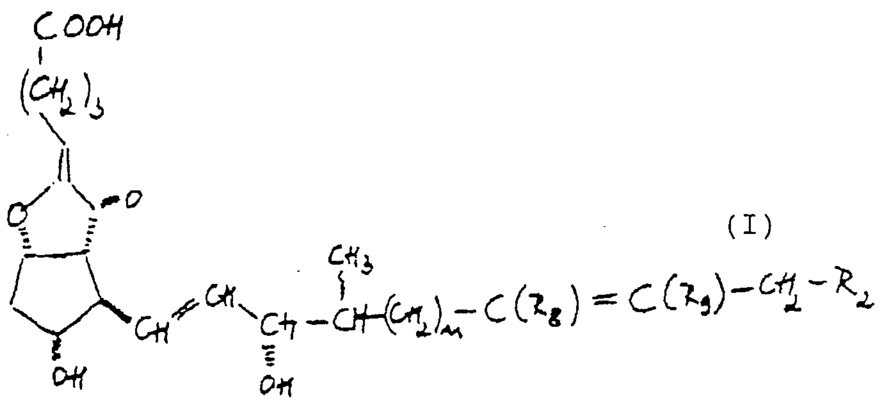

(I)

dans laquelle

| | |
|---|---|
| n | est égal à 1 ou à 2, |
| $R^2$ | représente l'hydrogène dans le cas où n est égal à 2, ou un radical méthyle dans le cas où n est égal à 1, et |
| $R^8$ et $R^9$ | forment ensemble une liaison ou représentent chacun l'hydrogène, ainsi que les sels qu'elles forment avec des bases acceptables du point de vue physiologique. |

2. Médicament constitué d'un ou de plusieurs composés selon la revendication 1 et d'adjuvants et excipients usuels.

3. Procédé pour préparer des oxo-7 prostacyclines de formule générale I, procédé caractérisé en ce qu'on oxyde au moyen de dioxyde de sélénium, de manière connue, un composé répondant à la formule générale II :

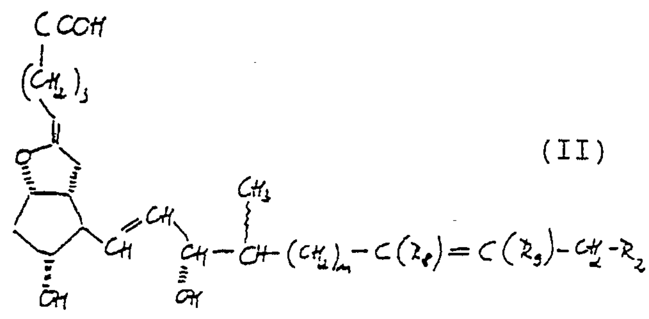

(II)

dans laquelle n, $R^2$, $R^8$ et $R^9$ ont les significations qui leur ont été données ci-dessus.